# EUROPEAN PATENT SPECIFICATION

(11) **EP 2 085 057 B1**
(45) Date of publication and mention of the grant of the patent: **12.10.2016**
(21) Application number: 07816578.4
(22) Date of filing: 16.10.2007
(51) Int. Cl.: A61L 31/10, A61L 31/16

(54) **VESSEL STENT WITH MULTI DRUG-COATINGS**
GEFÄSSSTENT MIT MEHRFACHER ARZNEIMITTELBESCHICHTUNG
STENT À PLUSIEURS COUCHES DE MÉDICAMENTS

(30) Priority: 20.11.2006 CN 200620148126 U; 28.12.2006 CN 200620166479 U
(43) Date of publication of application: 05.08.2009
(73) Proprietor: Lepu Medical Technology (Beijing) CO., LTD., Beijing 102200 (CN)
(72) Inventor: YU, Zhanjiang, Beijing 102200 (CN)
(74) Representative: Hoffmann Eitle
(86) International application number: PCT/CN2007/002964
(87) International publication number: WO 2008/061431

(56) References cited:
- WO-A-2005/089825
- WO-A2-2006/020742
- CN-A- 1 355 005
- CN-A- 1 465 410
- CN-A- 1 806 857
- CN-Y- 200 970 281
- CN-Y- 200 980 747
- CN-Y- 200 980 748
- CN-Y- 200 980 749
- CN-Y- 200 980 750
- HECTOR M GARCIA-GARCIA, SOPHIA VAINA, KEIICHI TSUCHIDA, PATRICK W SERRUYS: "Drug-eluting stens", ARCHIVOS DE CARDIOLOGIA DE MEXICO, vol. 76, no. 3, September 2006 (2006-09), pages 297-319, XP002701036,
- YOUNG JOON HONG, MYUNG HO JEONG: "New drug-eluting stents", KOREAN CIRCULATION J., vol. 35, 2005, pages 197-205, XP002701037,
- AOKI J ET AL: "Endothelial Progenitor Cell Capture by Stents Coated With Antibody Against CD34", JOURNAL OF THE AMERICAN COLLEGE OF CARDIOLOGY, ELSEVIER, NEW YORK, NY, US, vol. 45, no. 10, 17 May 2005 (2005-05-17), pages 1574-1579, XP027719658, ISSN: 0735-1097 [retrieved on 2005-05-17]
- ZHANJIANG YU ET AL.: "Accelerated endothelialization with a polymer-free sirolimus-eluting antibody-coated stent", J. MATER. SCI.: MATER. MED., 23 August 2013 (2013-08-23), DOI: 10.1007/s10856-013-5009-z
- YU ZHAN-JIANG ET AL.: "Effective adsorption of functional biological macromolecules on stainless steel surface with micro/nanoporous texture", ACTA PHYSICO-CHIMICA SINICA, vol. 29, 8 May 2013 (2013-05-08), pages 1595-1602,

## Description

### [TECHNICAL FIELD]

The present invention belongs to the field of medical devices and particularly relates to a vessel stent with multi drug-coatings covered with multiple layers of different drugs on the surface of the vessel stent or both the inside and outside surfaces of the vessel stent.

### [BACKGROUND ART]

Since Sigwart, etc. applied intravascular metal stent to coronary artery the first time in 1987, it provided a good way to treat diseases that block vessels. However, the vessel stent restenosis remains the main reason that influences the effect of percutaneous coronary interventional treatment (PCI). Research has confirmed that the main reason that causes the vessel in-stent restenosis is a series of reactions, including the formation of thrombus induced after stent or saccule hurts vessel, the inflammatory reaction of cells, the migration and proliferation of smooth muscle cells and the flexibility recovery of vascular tissue happening in the process of implanting vessel stent.

To overcome the vessel in-stent restenosis, drug-eluting stent has shown unparalleled superiority in anti-stenosis performance. The existing drug-eluting stent mainly composes of a metal stent body, a carrier of drugs and drugs. In general, the drugs for resisting the vessel in-stent restenosis are coated on the surface of stent with the help of the carrier of drugs. However, the drug-coatings are all single coating, which causes no breakthrough in multiple links including coating area ,coating layers and drug compatibility or prevention of the happening of the vessel in-stent restenosis, thereby can not effectively control the release of drugs at different phases of endothelial repair and can not bring about good effect of treatment. An example of a drug-eluting stent is taught by Young Joon Hong et al., Korean Circulation J., 2005, p. 197-205.

### [CONTENTS OF THE INVENTION]

The object of the present invention is to provide a vessel stent with multi drug-coatings with more reasonable coating area and coating layers, drug compatibility, which can prevent the multiple links of the happening of restenosis, control the release of drugs at different phases of endothelial repair and has better effect of treatment.

The solution to achieve the object of the present invention is as follows:
a vessel stent with multi drug-coatings comprises a stent body and active drugs, characterized in that a portion of surface or whole surface of the stent body is covered with at least one kind and at least one layer of drug coating as defined in claim 1.

The active drugs is dissolved in the solution of biodegradable polymeric materials or non-biodegradable polymeric materials; the biodegradable polymeric materials comprise one of homopolymer or copolymer of glycolide, lactide and ε-caprolactone, and copolymer formed by coplymerizing one of homopolymer or copolymer of glycolide, lactide and ε-caprolactone with multi-functional group of amino acids, polylactic acid, chitin, chitosan and collagen; the non-biodegradable polymeric materials comprise poly butyl methacrylate, polyethylene vinyl acetate copolymer, ethylene or vinyl acetate copolymer, polypropylene or polyacrylonitrile copolymer, poly-ε caprolactone.

The active drugs may include anti-inflammatory immunosuppressive agents, anti-proliferative drugs, anti-cell migration drugs, endothelialization of coronary drugs and polypeptide drugs. The anti-inflammatory immunosuppressive agents include sirolimus, tacrolimus, everolimus, immunosuppressive agents ABT-578, dexamethasone and mizoribine; the anti-proliferative drugs include rapamycin, paclitaxel, actinomycin, angiopeptim, vincristine and their derivatives, statins drugs, 2-chlorodeoxyadenosin, arsenic trioxide (As₂O₃) and ribozyme; the anti-cell migration drugs include batimastat, halofuginone, C-protease inhibitor and probucol; the coronary endothelialization drugs include endothelial growth factor, estradiols, penicillamine cyclopeptides, monoclonal antibody CD133 and its fragments, monoclonal antibody CD31 and its fragments, monoclonal antibody CD34 and its fragments and nucleic acid drugs.

Monoclonal antibody CD34 and its fragments acting as coronary endothelialization drugs are arranged on the inside surface of the stent body and rapamycin acting as drug resistant to proliferation of smooth muscle cells r is arranged on the outside surface of the stent body (1). The rapamycin drug is dissolved in the acetone and tetrahydrofuran solution of the non-biodegradable polymeric materials including polybutyl methacrylate, polyethylene vinyl acetate copolymer their equally mixedmixture. The rapamycin drug may also be dissolved in the acetone and tetrahydrofuran solution of one of the biodegradable polymeric materials including homopolymer or copolymer of glycolide, lactide and ε-caprolactone, and copolymer formed by coplymerizing one of homopolymer or copolymer of glycolide, lactide and ε-caprolactone with multi-functional group of amino acids, polylactic acid, chitin, chitosan and collagen.

Rapamycin acting as drug resistant to proliferation of smooth muscle cells is arranged on the outside surface of the stent body and monoclonal antibody CD34 and its fragments acting as coronary endothelialization drugs may be arranged on the outside surface of thesaidrapamycin. The said rapamycin is dissolved in the acetone and tetrahydrofuran solution of the non-biodegradable polymeric materials including polybutyl methacrylate, polyethylene vinyl acetate copolymer their equally mixed mixture, or dissolved in one of the biodegradable polymeric materials including homopolymer or copolymer of glycolide, lactide and ε-caprolactone, and copolymer formed by coplymerizing one of homopolymer or copolymer of glycolide, lactide and ε-caprolactone with multiple-functional group of amino acids, polylactic acid, chitin, chitosan and collagen.Same size holes with multiplecrystal phase structure are prepared on the surface of the stent body by chemical corrosion, electrochemical corrosion, anodic oxidation, micro-arc oxidation or micro-arc nitridation.

The outside surface of the stent body with holes is covered with rapamycin acting as drug resistant to proliferation of smooth muscle cells. The said rapamycin drug is dissolved in the solution of biodegradable polymeric materials or non-biodegradable polymeric materials, or directly dissolved in organic solvents and then is coated on the outside surface of the stent body.

The inside surface of the stent body with holes is embedded with the drug that captures vascular endothelial progenitor cells and promotes the endothelialization of the surface of the stent.

The drug that captures vascular endothelial progenitor cells and promotes the endothelialization of the surface of the stent includes endothelial growth factor, estradiols, penicillamine cyclopeptides

(cyclo-GpenGRGDSPCA), monoclonal antibody CD34 and its fragments, monoclonal antibody CD31 and its fragments, monoclonal antibody CD 133 and its fragments and nucleic acid drugs. Preferably, the inside surface of the stent body 1 with holes 101 is embedded with monoclonal antibody CD34 and its fragments or penicillamine cyclopeptides 203.

The beneficial effects of the present invention include:
1. The surface of the bare stent is covered with multiple layers of different drugs or the inside and outside surfaces are covered with different drugs, which can not only speed up endothelialization of coronary, but also resist cell proliferation, resist the migration of smooth muscle cells, reduce the formation of thrombus and the inflammatory reaction of cells and recover the flexibility of vascular tissue.
2. Two or more kinds of drugs acting at different links are coated on the same stent, which play the role of co-resisting the vessel in-stent restenosis by multiple drugs and resist the different pathological phases of the in-stent restenosis.
3. Multi drug-coatings have good anti-inflammatory effect and can resist the proliferation of cells, resist the migration of smooth muscle cells, speed up endothelialization of coronary, prevent multiple links of the happening of restenosis and resist the release of drugs at different phases of endothelial repair.
4. Drugs resistant to proliferation of smooth muscle cells and coronary endothelialization drug antibody are coated on the stent at the same time and the well- combined drug coating can solve the problems of the vessel in-stent restenosis and the stent later period thrombus effectively,make the use more secure and bring better effect of treatment.
5. On the surface of the bare stent same size holes with poly crystalline phases structure are prepared on the surface of the equipment body by chemical corrosion, electrochemical corrosion, anodic oxidation, micro-arc oxidation or micro-arc nitridation. The release of the drugs resistant to proliferation of smooth muscle cells on the outside surface can be controlled through the utilization of the size and depth of the holes. The coronary endothelialization drugs can be fixed through the utilization of the electrical property of the stent and the size and depth of the holes. The two drugs acting at the different links are assembled at a stent platform, through which the advantages of many kinds of drugs are shown and the problems of inflammatory and thrombus that may be induced by the carrier of prior drugs stent can be avoided at the same time.

### [DESCRIPTION OF FIGURES]

Fig. 1 illustrates cross-sectional cutaway view of the structure diagram of example 1 of the invention;
Fig. 2 illustrates cross-sectional cutaway view of the structure diagram of example 2 of the invention;
Fig. 3 illustrates cross-sectional cutaway view of the structure diagram of example 3 of the invention;
Fig. 4 illustrates cross-sectional cutaway view of the structure diagram of example 4 of the invention;
Fig. 5 illustrates the scanning electron microscope view of the stent body with holes of the invention;
Fig. 6 illustrates the scanning electron microscope view of the entire stent of the invention.

### [DETAILED MODE OF CARRYING OUT THE INVENTION]

The following examples are used to describe the present invention rather than limit the scope of the present invention.

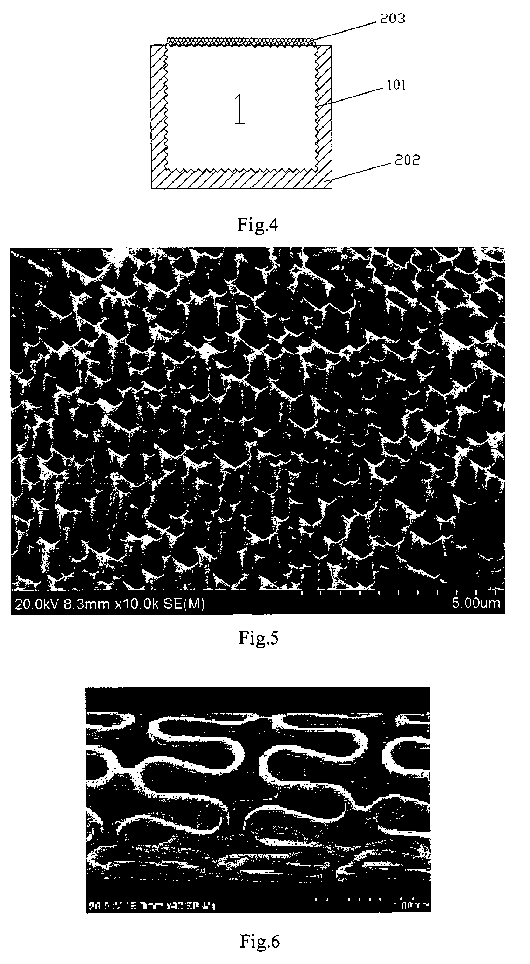

A vessel stent with multi drug-coatings including a stent body 1 and active drugs 2, and metal materials such as 316L stainless steel, cobalt based alloy and nitinol may be selected for the stent body 1. Fig. 6 is the vertical view of the stent body with holes of the present invention. On the stent body 1 there are holes formed through electrochemical corrosion and a portion of surface or whole surface of the stent body 1 is covered with one or more and at least one layer active drugs 2 coatings. The active drugs 2 are dissolved in solution of biodegradable polymeric materials or non-biodegradable polymeric materials, which is then coated on the stent body 1 through coating methods such as spraying, dip-coating, roller coating, brush coating, sputtering and plasma polymerization to form the drug-coatings.

The biodegradable polymeric materials comprise one of homopolymer or copolymer of glycolide, lactide and ε-caprolactone, and copolymer formed by coplymerizing one of homopolymer or copolymer of glycolide, lactide and ε-caprolactone with multi-functional group of amino acids, polylactic acid, chitin, chitosan and collagen.

The non-biodegradable polymeric materials comprise polybutyl methacrylate, polyethylene vinyl acetate copolymer, EVA, polypropylene or polyacrylonitrile copolymer, poly-ε caprolactone.

The active drugs 2 are monoclonal antibody CD34 or ariginine-glycerine-aspartic acid (RGD) polypeptide and rapamycin. Research and clinical analysis have confirmed that rapamycin drug 202 is an T cell inhibitor, as a kind of good anti-cell proliferation drug it can prevent T cell from transforming from G₁ period to S period and prevent Go period of B cell; monoclonal antibody CD34 and its fragments 201 can capture vascular endothelial progenitor cells (EPC) by combining antigen with antibody, speed up the differentiation of vascular endothelial progenitor cells on the surface of the stent into endothelial cells, and promote the repair of endothelium; for arginine-glycine-aspartic acid (RGD) cyclopeptides and modified products penicillamine cyclopeptides (cyclo-GpenGRGDSPCA) 203 that have higher affinity to integrin α5ß1 receptor than to αvß3 receptor, monoclonal antibody CD34 and its fragments 201 can capture more endothelial progenitor cells (EPCs) by combing the receptor with ligand to make the EPCs differentiate into vascular endothelial cells on the surface of the stent quickly and therefore promote the repair of endothelium.

Thus, the inside surface of the stent body 1 with holes 101 is embedded with monoclonal antibody CD34 and its fragments or arginine-glycine-aspartic acid (RGD) polypeptide drug 203, and the monoclonal antibody CD34 201 and its fragments or arginine-glycine-aspartic acid (RGD) polypeptide drug 203 can be dissolved in phosphate buffer (pH 7.2∼7.4) or carbonate buffer (pH 9.6) and then be directly embedded in the holes 101 on the inside surface of the stent body 1. The outside surface of the stent body 1 is sprayed with drug 202 resistant to proliferation of smooth muscle cells. which can be sprayed with the help of non-biodegradable polymeric materials or biodegradable polymeric materials or being dissolved in tetrahydrofuran solution directly (the weight percent is 0.2∼5%).

Hereafter the preferable embodiments will be given:

### Example 1

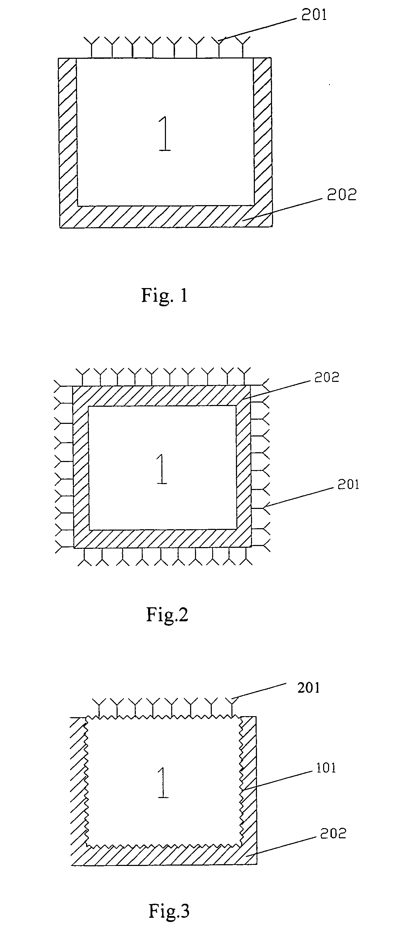

Fig. 1 illustrates cross-sectional cutaway view of the structure diagram of example 1 of the invention. The inside surface of the stent body 1 is covered with a layer of monoclonal antibody CD34 and its fragments 201, the outside of which is covered with rapamycin drug 202. The said rapamycin drug 202 is dissolved in the acetone and tetrahydrofuran solution of the non-biodegradable polymeric materials including polybutyl methacrylate PBMA, polyethylene vinyl acetate copolymer PEVA and their equally mixed mixture, or dissolved in the acetone and tetrahydrofuran solution of the biodegradablepolylactic acid or glycolic acid copolymer PLGA, or polylactic acid, and then sprayed or dig-coated on the outside surface of the stent body 1.

### Example 2

Fig. 2 illustrates cross-sectional cutaway view of the structure diagram of example 2 of the Invention. The whole surface of the stent body 1 is covered with a layer of rapamycin drug 202. On the outside surface of rapamycin drug 202 is covered with a layer of monoclonal antibody CD34 and its fragment drug 201. The rapamycin drug 202 is dissolved in the tetrahydrofuran solution of the non-biodegradable polybutyl methacrylate PBMA or the biodegradable polylactic acid or glycolic acid copolymer PLGA, and then sprayed or dig-coated on the outside surface of the stent body 1.

### Example 3

Fig. 3 illustrates cross-sectional cutaway view of the structure diagram of example 3 of the Invention. The inside surface of the stent body 1 with holes 101 is embedded with monoclonal antibody CD34 and its fragment drug 201, the outside surface of which is covered with rapamycin 202 acting as drug resistant toproliferation of smooth muscle cells, rapamycin may be dissolved in the non-biodegradable polybutyl methacrylate of 0.2-5% (weight percent), or tetrahydrofuran solution of the biodegradable polylactic acid-polyglycolic acid copolymer of 0.5-10% (weight percent), or directly dissolved in the tetrahydrofuran solution of 0.5-10% (weight percent), and then sprayed on the outside surface of the stent body 1.

### Example 4

Fig. 4 illustrates cross-sectional cutaway view of the structure of the example 4 of the invention. The inside surface of the stent body 1 with holes 101 is embedded with arginine-glycine-aspartic acid (RGD) polypeptide drug 203. The polypeptide drug 203 is dissolved in phosphate buffer (pH 7.2∼7.4) or carbonate buffer (pH 9.6) and then embedded on the inside surface of the stent body 1. The outside surface of the stent body 1 is covered with rapamycin 202 acting as drug resistant toproliferation of smooth muscle cells which may be dissolved in the solution of non-biodegradable 1 polybutyl methacrylate of 0.2-5% (weight percent) or the tetrahydrofuran solution of biodegradable polylactic acid-polyglycolic acid copolymer of 0.5-10% (weight percent), or directly dissolved in the tetrahydrofuran solution of 0.5-10% (weight percent), and then sprayed on the outside surface of the stent body 1.

While the invention has been described in terms of various specific embodiments and general description, it will be obvious to those skilled in the art that certain modification or improvement should be made to the invention as described without departing from the scope of the invention.

### [INDUSTRIAL APPLICABILITY]

Basen on the prior vessel stent, the present invention utilizes physical or chemical method to corrode and form holes. According to their properties, the active drugs are dissolved in the solution of biodegradable polymeric materials or non-biodegradable polymeric materials, or directly dissolved in organic solvents (tetrahydrofuran, acetone, chloroform, etc.) or phosphate buffer (pH 7.2∼7.4) or carbonate buffer (pH 9.6), which can be coated on the stent body through drug coating methods such as spraying, dip-coating, roller coating, brush coating, sputtering and plasma polymerization to form the drug-coatings. The active drug monoclonal antibody CD34 and its fragments can capture vascular endothelial progenitor cells by combing antigen with antibody, speed up the differentiation of vascular endothelial progenitor cells on the surface of the stent into endothelial cells and promote the repair of endothelium. As a good anti-cell proliferation drug, the active drug, rapamycin can prevent T cell from transforming from G₁ period to S period and prevent Go period of B cell; arginine-glycine-aspartic acid (RGD) cyclopeptides and its modified products-penicillamine cyclopeptides (cyclo-GpenGRGDSPCA) can capture more endothelial progenitor cells (EPCs) by combing the receptor with ligand to make the EPCs differentiate into vascular endothelial cells on the surface of the stent quickly and therefore promote the repair of endothelium. Thus, by the way of embedding the drugs mentioned above on the surface of the stent body with holes and utilizing the size and depth, the speed of release of drugs can be effectively controlled. At the same time, the coronary endothelialization drugs can be fixed through the utilization of the electrical property of the stent and the size and depth of the holes. The two drugs acting at the different links are assembled at a stent platform, through which the advantages of many kinds of drugs are shown, the problems of inflammatory and thrombus that may be induced by the carrier of prior drugs stent can be avoided, and the rate of formation of thrombus and the incidence of the disease of in-stent restenosis can be reduced effectively at the same time.

## Claims

1. A vessel stent with multi drug-coatings including a stent body (1) and active drugs (2), **characterized in that**, a portion of surface or whole surface of the stent body (1) is covered with at least one kind and at least one layer of active drugs (2) coating wherein monoclonal antibody CD34 or arginine-glycerine-aspartic acid (RGD) polypeptide drug is arranged on the inside of the stent body and rapamycin is arranged on the outside surface of the stent body.

2. The vessel stent with multi drug-coatings according to claim 1, **characterized in that**, the active drugs (2) are dissolved in the solution of biodegradable or non-biodegradable polymeric materials; the said biodegradable polymeric materials comprise one of homopolymer or copolymer of glycolide, lactide and [epsilon]-caprolactone, and copolymer formed by coplymerizing one of homopolymer or copolymer of glycolide, lactide and [epsilon]-caprolactone with multi-functional group of amino acids, polylactic acid, chitin, chitosan and collagen; the said non-biodegradable polymeric materials comprise polybutyl methacrylate, polyethylene vinyl acetate copolymer, ethylene or vinyl acetate copolymer, polypropylene or polyacrylonitrile copolymer, poly-[epsilon] caprolactone.

3. The vessel stent with multi drug-coatings according to claim 1, **characterized in that**, the active drugs (2) include anti-inflammatory immunosuppressive agents, anti-proliferative drugs, anti-cell migration drugs, endothelialization of coronary drugs and polypeptide drugs; the said anti-inflammatory immunosuppressive agents include sirolimus, tacrolimus, everolimus, immunosuppressive agents ABT-578, dexamethasone and mizoribine; the anti-proliferative drugs include paclitaxel, actinomycin, angiopeptim, vincristine and their derivatives, statins drugs, 2-chlorodeoxyadenosin, arsenic trioxide (As₂O₃) and ribozyme; the anti-cell migration drugs include batimastat, halofuginone, C-protease inhibitor and probucol; the coronary endothelialization drugs include endothelial growth factor, estradiols, penicillamine cyclopeptides, monoclonal antibody CD 133, monoclonal antibody CD31, and nucleic acid drugs.

4. The vessel stent with multi drug-coatings according to claim 1 or 2 or 3, **characterized in that**, monoclonal antibody CD34 (201) acting as coronary endothelialization drugs are arranged on the inside surface of the stent body (1) and drugs resistant to proliferation of smooth muscle cells is arranged on the outside surface of the stent body (1); the said rapamycin drug (202) is dissolved in the acetone and tetrahydrofuran solution of the non-biodegradable polymeric materials including polybutyl methacrylate, polyethylene vinyl acetate copolymer and their equally mixed mixture, or dissolved in one of the biodegradable polymeric materials including homopolymer or copolymer of glycolide, lactide and [epsilon]-caprolactone, and copolymer formed by coplymerizing one of homopolymer or copolymer of glycolide, lactide and [epsilon]-caprolactone with multi-functional group of amino acids, polylactic acid, chitin, chitosan and collagen.

5. The vessel stent with multi drug-coatings according to claim 1 or 2 or 3, **characterized in that**, rapamycin (202) acting as a drug resistant to proliferation of smooth muscle cells is arranged on the outside surface of the stent body (1) and monoclonal antibody CD34 (201) acting as coronary endothelialization drugs are arranged on the outside surface of the stent body (1); rapamycin (202) is dissolved in the acetone and tetrahydrofuran solution of the non-biodegradable polymeric materials including polybutyl methacrylate, polyethylene vinyl acetate copolymer and their equally mixed mixture, or dissolved in one of the biodegradable polymeric materials including homopolymer or copolymer of glycolide, lactide and [epsilon]-caprolactone, and copolymer formed by coplymerizing one of homopolymer or copolymer of glycolide, lactide and [epsilon]-caprolactone with multi-functional group of amino acids, polylactic acid, chitin, chitosan and collagen.

6. The vessel stent with multi drug-coatings according to claim 1, **characterized in that**, same size holes (101) with multicrystal phase structure are prepared on the surface of the stent body (1) by chemical corrosion, electrochemical corrosion, anodic oxidation, micro-arc oxidation or micro-arc nitridation.

7. The vessel stent with multi drug-coatings according to claim 6,**characterized in that**, the outside surface of the stent body (1) with holes (101) is covered with rapamycin (202) acting as drug resistant to proliferation of smooth muscle cells ; rapamycin (202) is dissolved in the solution of biodegradable or non-biodegradable polymeric materials, or directly dissolved in organic solvents and then is coated on the outside surface of the stent body (1).

8. The vessel stent with multi drug-coatings according to claim 6, **characterized in that**, the inside surface of the stent body (1) with holes (101) is embedded with drugs that capture vascular endothelial progenitor cells and promote the endothelialization of the the stent surface.

9. The vessel stent with multi drug-coatings according to claim 8, **characterized in that**, the drugs that capture vascular endothelial progenitor cells and promote the endothelialization of the the stent surface include endothelial growth factor, estradiols, penicillamine cyclopeptides, monoclonal antibody CD34, monoclonal antibody CD31, monoclonal antibody CD 133 and nucleic acid drugs.

10. The vessel stent with multi drug-coatings according to claim 9, **characterized in that**, the inside surface of the stent body (1) with holes (101) is embedded with monoclonal antibody CD34 (201) or penicillamine cyclopeptides (203).

## Patentansprüche

1. Gefäß-Stent mit mehrfachen Arzneistoff-Beschichtungen, welcher einen Stentkörper (1) und aktive Arzneistoffe (2) einschließt, **dadurch gekennzeichnet, dass** ein Abschnitt der Oberfläche oder die gesamte Oberfläche des Stentkörpers (1) mit wenigstens einer Art und wenigstens einer Schicht aus aktiver Arzneistoff- (2) Beschichtung bedeckt ist, wobei monoklonaler Antikörper CD34 oder Arginin-Glycerin-Asparaginsäure- (RGD-) Polypeptid-Arzneistoff auf der Innenseite des Stentkörpers angeordnet ist und Rapamycin auf der äußeren Oberfläche des Stentkörpers angeordnet ist.

2. Gefäß-Stent mit mehrfachen Arzneistoff-Beschichtungen gemäß Anspruch 1, **dadurch gekennzeichnet, dass** die aktiven Arzneistoffe (2) in der Lösung aus biologisch abbaubaren oder nicht biologisch abbaubaren Polymermaterialien gelöst sind; die biologisch abbaubaren Polymermaterialien eines von einem Homopolymer oder Copolymer von Glycolid, Lactid und [Epsilon]-Caprolacton, und Copolymer, gebildet durch Copolymerisieren eines von einem Homopolymer oder Copolymer von Glycolid, Lactid und [Epsilon]-Caprolacton mit einer multifunktionalen Gruppe von Aminosäuren, Polymilchsäure, Chitin, Chitosan und Kollagen, umfassen; die nicht biologisch abbaubaren Polymermaterialien Polybutylmethacrylat, Polyethylenvinylacetat-Copolymer, Ethylen- oder Vinylacetat-Copolymer, Polypropylen- oder Polyacrylnitril-Copolymer, Poly-[Epsilon]-Caprolacton umfassen.

3. Gefäß-Stent mit mehrfachen Arzneistoff-Beschichtungen gemäß Anspruch 1, **dadurch gekennzeichnet, dass** die aktiven Arzneistoffe (2) anti-inflammatorische immunsuppressive Mittel, anti-proliferative Arzneistoffe, Anti-Zellmigrations-Arzneistoffe, koronare Endothelialisierungs-Arzneistoffe und Polypeptid-Arzneistoffe einschließen; die anti-inflammatorischen immunsuppressiven Mittel Sirolimus, Tacrolimus, Everolimus, immunsuppressive Mittel ABT-578, Dexamethason und Mizoribin einschließen; die antiproliferativen Arzneistoffe Paclitaxel, Actinomycin, Angiopeptin, Vincristin und deren Derivate, Statin-Arzneistoffe, 2-Chlordesoxyadenosin, Arsentrioxid (As₂O₃) und Ribozym einschließen; die Anti-Zellmigrations-Arzneistoffe Batimastat, Halofuginon, C-Protease-Inhibitor und Probucol einschließen; die koronaren Endothelialisierungs-Arzneistoffe endothelialen Wachstumsfaktor, Estradiole, Penicillamin-Cyclopeptide, monoklonalen Antikörper CD 133, monoklonalen Antikörper CD31 und Nukleinsäure-Arzneistoffe einschließen.

4. Gefäß-Stent mit mehrfachen Arzneistoff-Beschichtungen gemäß Anspruch 1 oder 2 oder 3, **dadurch gekennzeichnet, dass** monoklonale Antikörper CD34 (201), die als koronare Endothelialisierungs-Arzneistoffe wirken, auf der inneren Oberfläche des Stentkörpers (1) angeordnet sind und gegen die Proliferation glatter Muskelzellen resistente Arzneistoffe auf der äußeren Oberfläche des Stentkörpers (1) angeordnet sind; wobei der Rapamycin-Arzneistoff (202) in der Aceton- und Tetrahydrofuran-Lösung der nicht biologisch abbaubaren Polymermaterialien, einschließlich Polybutylmethacrylat, Polyethylenvinylacetat-Copolymer und gleichmäßig gemischten Gemischen davon, gelöst ist, oder in einem der biologisch abbaubaren Polymermaterialien, einschließlich Homopolymer oder Copolymer von Glycolid, Lactid und [Epsilon]-Caprolacton, und Copolymer, gebildet durch Copolymerisieren eines von einem Homopolymer oder Copolymer von Glycolid, Lactid und [Epsilon]-Caprolacton mit einer multifunktionalen Gruppe von Aminosäuren, Polymilchsäure, Chitin, Chitosan und Kollagen, gelöst ist.

5. Gefäß-Stent mit mehrfachen Arzneistoff-Beschichtungen gemäß Anspruch 1 oder 2 oder 3, **dadurch gekennzeichnet, dass** Rapamycin (202), das als Arzneistoff wirkt, der gegen die Proliferation glatter Muskelzellen resistent ist, auf der äußeren Oberfläche des Stentkörpers (1) angeordnet ist, und monoklonale Antikörper CD34 (201), die als koronare Endothelialisierungs-Arzneistoffe wirken, auf der äußeren Oberfläche des Stentkörpers (1) angeordnet sind; Rapamycin (202) in der Aceton- und Tetrahydrofuran-Lösung der nicht biologisch abbaubaren Polymermaterialien, einschließlich Polybutylmethacrylat, Polyethylenvinylacetat-Copolymer und gleichmäßig gemischten Gemischen davon, gelöst ist, oder in einem der biologisch abbaubaren Polymermaterialien, einschließlich Homopolymer oder Copolymer von Glycolid, Lactid und [Epsilon]-Caprolacton, und Copolymer, gebildet durch Copolymerisieren eines von Homopolymer oder Copolymer von Glycolid, Lactid und [Epsilon]-Caprolacton mit einer multifunktionalen Gruppe von Aminosäuren, Polymilchsäure, Chitin, Chitosan und Kollagen, gelöst ist.

6. Gefäß-Stent mit mehrfachen Arzneistoff-Beschichtungen gemäß Anspruch 1, **dadurch gekennzeichnet, dass** Löcher (101) der gleichen Größe mit multikristalliner Phasen-Struktur durch chemische Korrosion, elektrochemische Korrosion, anodische Oxidation, Mikrolichtbogen-Oxidation oder Mikrolichtbogen-Nitrierung in der Oberfläche des Stentkörpers (1) hergestellt werden.

7. Gefäß-Stent mit mehrfachen Arzneistoff-Beschichtungen gemäß Anspruch 6, **dadurch gekennzeichnet, dass** die äußere Oberfläche des Stentkörpers (1) mit Löchern (101) mit Rapamycin (202) bedeckt ist, das als Arzneistoff wirkt, der gegen die Proliferation glatter Muskelzellen resistent ist; Rapamycin (202) in der Lösung von biologisch abbaubaren oder nicht biologisch abbaubaren Polymermaterialien gelöst ist, oder direkt in organischen Lösungsmitteln gelöst ist und dann auf die äußere Oberfläche des Stentkörpers (1) aufgeschichtet wird.

8. Gefäß-Stent mit mehrfachen Arzneistoff-Beschichtungen gemäß Anspruch 6, **dadurch gekennzeichnet, dass** Arzneistoffe in die innere Oberfläche des Stentkörpers (1) mit Löchern (101) eingebettet sind, die vaskuläre Endothel-Vorläuferzellen einfangen und die Endothelialisierung der Stentoberfläche fördern.

9. Gefäß-Stent mit mehrfachen Arzneistoff-Beschichtungen gemäß Anspruch 8, **dadurch gekennzeichnet, dass** die Arzneistoffe, die vaskuläre Endothel-Vorläuferzellen einfangen und die Endothelialisierung der Stentoberfläche fördern, endothelialen Wachstumsfaktor, Estradiole, Penicillamin-Cyclopeptide, monoklonalen Antikörper CD34, monoklonalen Antikörper CD31, monoklonalen Antikörper CD 133 und Nukleinsäure-Arzneistoffe einschließen.

10. Gefäß-Stent mit mehrfachen Arzneistoff-Beschichtungen gemäß Anspruch 9, **dadurch gekennzeichnet, dass** monoklonaler Antikörper CD34 (201) oder Penicillamin-Cyclopeptide (203) in die innere Oberfläche des Stentkörpers (1) mit Löchern (101) eingebettet sind.

## Revendications

1. Stent de vaisseau à plusieurs revêtements de médicaments incluant un corps de stent (1) et des médicaments actifs (2), **caractérisé en ce qu'**une portion de surface ou la surface entière du corps de stent (1) est recouverte d'au moins une sorte et d'au moins une couche de revêtement de médicaments actifs (2), dans lequel l'anticorps monoclonal CD34 ou le médicament polypeptidique arginine-glycérine-acide aspartique (RGD) est arrangé à l'intérieur du corps de stent, et la rapamycine est arrangée sur la surface extérieure du corps de stent.

2. Stent de vaisseau à plusieurs revêtements de médicaments selon la revendication 1, **caractérisé en ce que** les médicaments actifs (2) sont dissous dans la solution de matériaux polymères biodégradables ou non-biodégradables; lesdits matériaux polymères biodégradables comprennent un d'un homopolymère ou copolymère de glycolide, de lactide et d'[epsilon]-caprolactone, et d'un copolymère formé en copolymérisant un d'un homopolymère ou copolymère de glycolide, de lactide et d'[epsilon]-caprolactone avec un groupe multi-fonctionnel d'acides aminés, d'acide polylactique, de chitine, de chitosan et de collagène ; lesdits matériaux polymères non-biodégradables comprennent du méthacrylate de polybutyle, un copolymère de polyéthylène-acétate de vinyle, un copolymère d'éthylène ou d'acétate de vinyle, un copolymère de polypropylène ou de polyacrylonitrile, de la poly-[epsilon] caprolactone.

3. Stent de vaisseau à plusieurs revêtements de médicaments selon la revendication 1, **caractérisé en ce que** les médicaments actifs (2) incluent des agents immunosuppresseurs anti-inflammatoires, des médicaments anti-prolifératifs, des médicaments anti-migration cellulaire, des médicaments d'endothélialisation coronaire et des médicaments polypeptidiques ; lesdits agents immunosuppresseurs anti-inflammatoires incluent le sirolimus, le tacrolimus, l'évérolimus, les agents immunosuppresseurs ABT-578, la dexaméthasone et la mizoribine ; les médicaments anti-prolifératifs incluent le paclitaxel, l'actinomycine, l'angiopeptine, la vincristine et leurs dérivés, des médicaments statines, la 2-chlorodéoxyadénosine, le trioxyde d'arsenic (As₂O₃) et le ribozyme ; les médicaments anti-migration cellulaire incluent le batimastat, l'halofuginone, l'inhibiteur de protéase C et le probucol ; les médicaments d'endothélialisation coronaire incluent le facteur de croissance endothéliale, les estradiols, la pénicillamine, les cyclopeptides, l'anticorps monoclonal CD 133, l'anticorps monoclonal CD31 et les médicaments d'acide nucléique.

4. Stent de vaisseau à plusieurs revêtements de médicaments selon la revendication 1 ou 2 ou 3, **caractérisé en ce que** l'anticorps monoclonal CD34 (201) agissant en tant que médicaments d'endothélialisation coronaire est arrangé sur la surface intérieure du corps de stent (1) et des médicaments résistant à la prolifération de cellules musculaires lisses sont arrangés sur la surface extérieure du corps de stent (1) ; ledit médicament rapamycine (202) est dissous dans la solution d'acétone et de tétrahydrofurane des matériaux polymères non-biodégradables incluant du méthacrylate de polybutyle, le copolymère de polyéthylène-acétate de vinyle et leur mélange également mélangé, ou dissous dans l'un des matériaux polymères biodégradables incluant un homopolymère ou copolymère de glycolide, de lactide et d'[epsilon]-caprolactone, et un copolymère formé en copolymérisant un d'un homopolymère ou copolymère de glycolide, de lactide et d'[epsilon]-caprolactone avec un groupe multi-fonctionnel d'acides aminés, d'acide polylactique, de chitine, de chitosan et de collagène.

5. Stent de vaisseau à plusieurs revêtements de médicaments selon la revendication 1 ou 2 ou 3, **caractérisé en ce que** la rapamycine (202) agissant en tant que médicament résistant à la prolifération de cellules musculaires lisses est arrangée sur la surface extérieure du corps de stent (1), et l'anticorps monoclonal CD34 (201) agissant en tant que médicaments d'endothélialisation coronaire est arrangé sur la surface extérieure du corps de stent (1) ; la rapamycine (202) est dissoute dans la solution d'acétone et de tétrahydrofurane des matériaux polymères non-biodégradables incluant le méthacrylate de polybutyle, un copolymère de polyéthylène ou d'acétate de vinyle et leur mélange également mélangé, ou dissoute dans l'un des matériaux polymères biodégradables incluant un homopolymère ou copolymère de glycolide, de lactide et d'[epsilon]-caprolactone, et un copolymère formé en copolymérisant un d'un homopolymère ou copolymère de glycolide, de lactide et d'[epsilon]-caprolactone avec un groupe multi-fonctionnel d'acides aminés, d'acide polylactique, de chitine, de chitosan et de collagène.

6. Stent de vaisseau à plusieurs revêtements de médicaments selon la revendication 1, **caractérisé en ce que**, des trous de même taille (101) avec une structure de phase multicristalline sont préparés sur la surface du corps de stent (1) par corrosion chimique, corrosion électrochimique, oxydation anodique, oxydation micro-arc ou nitruration micro arc.

7. Stent de vaisseau à plusieurs revêtements de médicaments selon la revendication 6, **caractérisé en ce que** la surface extérieure du corps de stent (1) avec trous (101) est recouverte avec de la rapamycine (202) agissant en tant que médicament résistant à la prolifération de cellules musculaires lisses; la rapamycine (202) est dissoute dans la solution de matériaux polymères biodégradables ou non-biodégradables, ou directement dissoute dans des solvants organiques, et est ensuite revêtue sur la surface extérieure du corps de stent (1).

8. Stent de vaisseau à plusieurs revêtements de médicaments selon la revendication 6, **caractérisé en ce que** la surface intérieure du corps de stent (1) avec trous (101) intègre des médicaments qui capturent des cellules progénitrices endothéliales vasculaires et favorisent l'endothélialisation de la surface de stent.

9. Stent de vaisseau à plusieurs revêtements de médicaments selon la revendication 8, **caractérisé en ce que** les médicaments qui capturent des cellules progénitrices endothéliales vasculaires et favorisent l'endothélialisation de la surface du stent incluent un facteur de croissance endothéliale, des estradiols, des cyclopeptides de pénicillamine, un anticorps monoclonal CD34, un anticorps monoclonal CD31, un anticorps monoclonal CD 133 et des médicaments d'acide nucléique.

10. Stent de vaisseau à plusieurs revêtements de médicaments selon la revendication 9, **caractérisé en ce que** la surface intérieure du corps de stent (1) avec trous (101) intègre un anticorps monoclonal CD34 (201) ou des cyclopeptides de pénicillamine (203).
